# EUROPEAN PATENT APPLICATION

(11) **EP 0 580 384 A1**
(43) Date of publication of application: **26.01.1994**
(21) Application number: 93305648.3
(22) Date of filing: 19.07.1993
(51) Int. Cl.: A61F 5/44, A61M 39/00, F16K 5/04

(54) **A flow control device**

(30) Priority: 20.07.1992 GB 9215371
(71) Applicant: SIMPLA PLASTICS LIMITED, Cardiff CF4 5WF (GB)
(72) Inventor: Karlsen, Sven, Belton, Doncaster (GB)
(74) Representative: Johnson, Terence Leslie

(57) **Abstract**

The invention relates to a tap for controlling flow of fluid such as urine along a conduit (2), comprising a tube (3) defining the conduit (2), a housing (4) in the tube, a rotatable barrel (5) in the housing (4) having a bore (6) alignable with the conduit (2), and being adapted in one position to close the tube (3) against urine flow through the conduit (2) and in a second position to open the tube (3) to urine flow with the conduit (2) and bore (6) being substantially aligned, and a handle (7) mounted on the barrel with a lost motion connection (8) whereby in both the open and closed positions the handle (7) lies closely adjacent the tube (2) and at an inclination to the longitudinal axis of the tube.

## Description

The invention relates to a flow control device such as a tap for controlling fluid flow along a conduit. The invention is particularly applicable to taps for use in urine drainage systems, for controlling flow of urine to a urine collection bag.

Urine drainage bags are often in the form of leg bags which collect urine from a catheterised patient and then feed that urine on to a larger bag such as an overnight or reservoir bag which might be free-standing on a frame on the floor, or may be on a frame hooked over the frame of the patient's bed. In either case, taps of this system are often left open for a relatively long period of time, say overnight, in order to ensure passage of the urine to the larger collection bag. Previous taps, whilst being satisfactory in their action of opening and closing a flowpath for urine nevertheless have a disadvantage, which is that when open an operating lever is often at substantially 90° to the flowpath. This means that the lever is able to be "snagged" by a passing person or can otherwise accidentally be closed, with uncomfortable consequences for the user.

It is an object of the invention to seek to mitigate these disadvantages.

According to a first aspect of the invention there is provided a tap for controlling fluid flow along a conduit, comprising a tube, a housing in the tube, a rotatable barrel in the housing having a bore alignable with the tube longitudinal axis and being adapted in one position to close the tube against fluid flow and in a second position to open the tube to fluid flow, a handle mounted on the barrel and a lost motion connection between the handle and barrel whereby in both the open and closed positions the handle lies closely adjacent the tube and at an inclination to the longitudinal axis of the tube.

The lost motion connection may comprise complementary parts of the handle and barrel and an abutment of one said part adapted to be engaged by a driving member of the other part. This provides for a relatively simple construction.

The complementary parts may comprise a boss of the barrel carrying the abutment, and a socket of the handle carrying the driving member. This provides for positive connection and operation.

This abutment may comprise a diametral bar of the boss and the driving member may comprise a segment-shaped step in the socket. This construction can be relatively readily provided using simple mould tools when the respective parts are moulded.

The step may have an angle of between 90° and 180°, suitably 145°; this provides an ample lost motion connection to achieve the desired result of the handle lying close to the tube.

The handle may comprise two substantially L-shaped handle parts which are mounted on the barrel and which are secured together in planes laterally displaced from the axis of rotation of the barrel. This provides for a hand grip on each side of the tube.

The handle parts may be secured together by complementary push-fit means. This provides a relatively simple construction and assembly.

The handle may comprise a substantially U-shaped member mounted on the barrel and the limbs of which are connected by a yoke. This is a relatively simple alternative construction, particularly where the limbs and yoke are secured together by complementary push-fit means.

In another embodiment the handle may be a substantially U-shaped member mounted on the barrel, and may terminate in free ends which are directed towards one another. This provides a relatively smooth contour of the tap.

In another modification, the handle may comprise a one piece handle moulded with a plurality, preferably 4, built-in hinge devices.

There may be a cap mounted on the end of the barrel opposite the end on which the handle is mounted. This provides a relatively simple way of closing off the barrel.

The limbs may terminate in free ends which lie substantially in a plane at a tangent to the outer surface of the tube. This again can provide a smooth contour.

The handle may comprise a single L-shaped member mounted on the barrel by one limb, and the other limb of the L-shaped member may terminate at its free end in a plane lying substantially in a plane at a tangent to the outer surface of the tube. This again provides an embodiment with a relatively smooth exterior.

The L-shaped member may also be mounted by one limb on the barrel, and the free end of the other limb may be directed towards the axis of rotation of the barrel. This provides a relatively compact structure.

The component parts of the tap may be moulded from plastic. This provides a relatively inexpensive yet repeatable constructional method.

According to a second aspect of the invention there is provided a urine drainage system, including a tap as hereinbefore defined.

Taps embodying the invention are hereinafter described, by way of example, with reference to the accompanying drawings.
Figs. 1A - Fig. 1D show respectively an exploded plan view of a first embodiment of a tap according to the invention showing its use, a plan view of the assembled tap, and end elevational views of the tap in the open and closed position.
Figs. 2A - 2D, 3A - 3D, 4A to 4D, 5A to 5D and 6A - 6D show views similar to those of Figs. 1A to 1D but respectively of second, third, fourth, fifth and sixth embodiments of a tap according to the invention;
Figs. 7A to 7C show a sequence of operations of the taps of Figs. 1 to 6D; and
Fig.8 is to an enlarged scale, an exploded elevational view of a seventh embodiment of tap according to the invention.

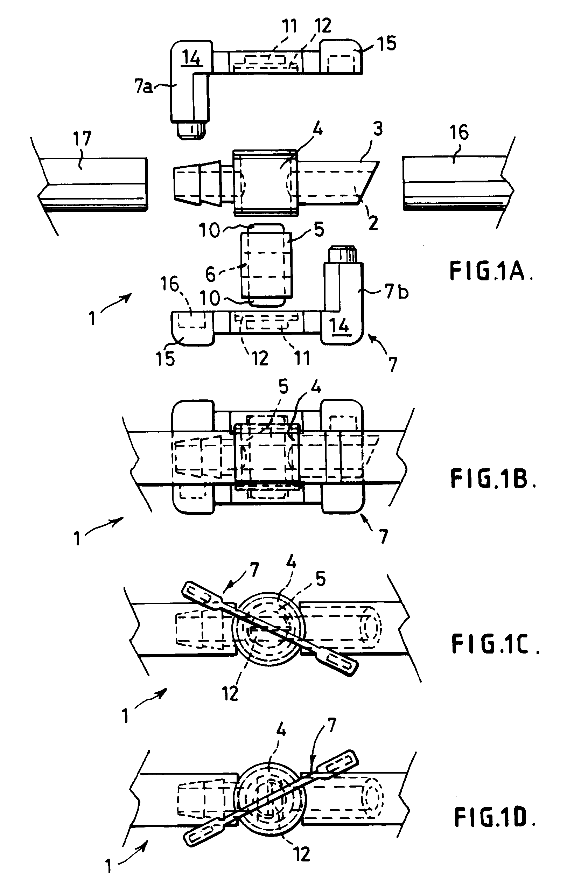

Referring to the drawings, in which like reference numerals are used to indicate like parts, there is shown in Figs 1A to 1D a tap for controlling flow of fluid such as urine along a conduit 2, comprising a tube 3 defining the conduit 2, a housing 4 in the tube, a rotatable barrel 5 in the housing 4 having a bore 6 alignable with the conduit 2, and being adapted in one position (Fig. 1D) to close the tube 3 against urine flow through the conduit 2 and in a second position (Fig. 1C) to open the tube 3 to urine flow with the conduit 2 and bore 6 being substantially aligned, and a handle 7 mounted on the barrel with a lost motion connection 8 whereby in both the open and closed positions the handle 7 lies closely adjacent the tube 2 and at an inclination to the longitudinal axis of the tube.

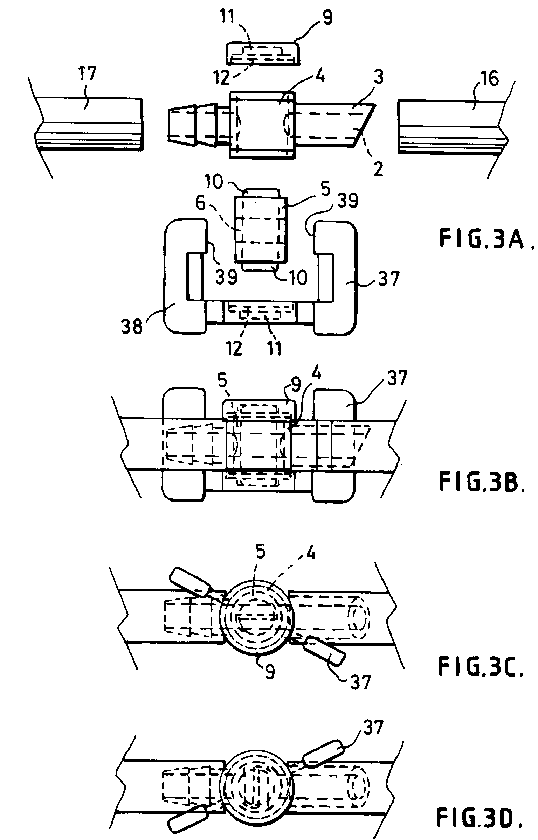

In each embodiment the housing 4 is an integral part of the tube 3, and they are formed as by moulding from plastic material, as is the barrel itself, the handle and an end cap 9 (Figs. 3A - 6A).

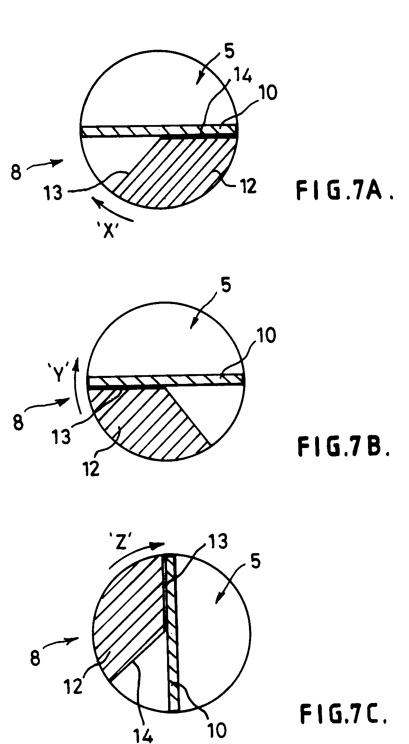

The barrel 5 carries the bore 6 which is a through bore, the body of the barrel being in close fit in the housing 4. The barrel 5 projects from the housing 4 at each end, which ends each have a boss, abutment or bar 10 which stands proud of the respective end. The bar 10 is complementary to a driving member of the handle 7, which in Figs. 1A - 1D comprises two handle parts 7a, 7b of L-shape, each of which is a push-fit on the end of the barrel 5 so that the respective end is received in a socket 11 of the handle part 7a or 7b and which handle parts each have in their socket 11 the driving member 12 which is in the form of a 145° segment of a circle in each socket 11 and forming a part of the base thereof with a surface 13 being directed away from the bar 10 in the first or open position (Figs. 1C, 7A). The bar 10 lies adjacent the segment in the socket.

The L-shaped handle parts 7a and 7b form cross or transverse members and are reversed so that the shorter leg 14 of one 7a is received as by being received with a push-fit in the longer limb 15 of the other part 7b whereby to provide a rectangular handle one opposite pair of sides being mounted on the barrel 5, and the other opposite pair of sides lying closely adjacent the tube 3 on diametrically opposite sides thereof as viewed in Figs. 1C and 1D. Thus the projecting sides of the handle 7 are not projecting from the tube a distance in order to be inadvertently operated. This is achieved in that the complementary bar and segment form a lost motion connection of the handle and barrel.

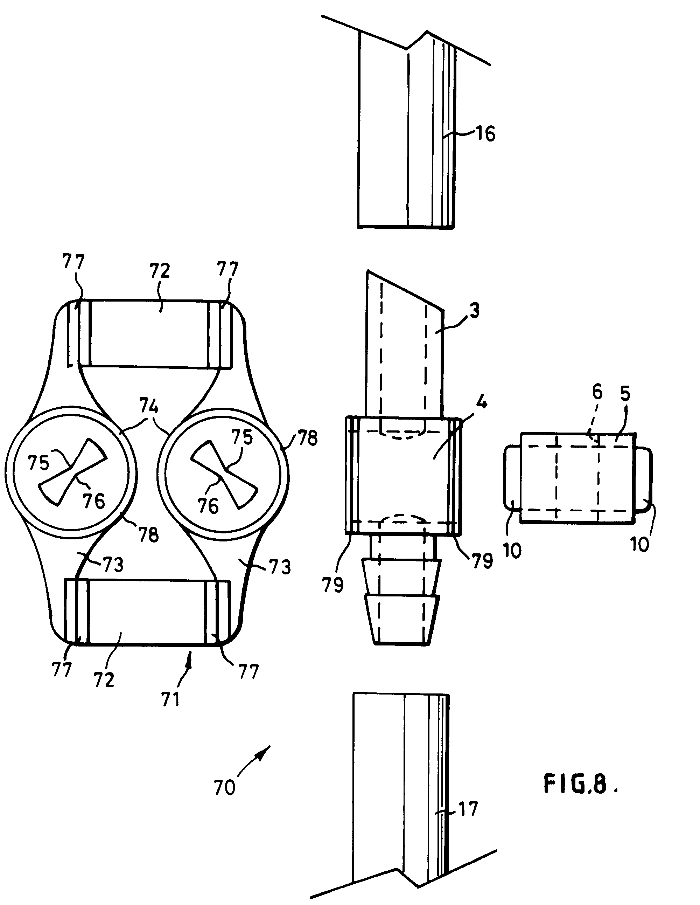

Fig.8 shows a further embodiment of tap 70, in exploded form, and to a larger scale than that of Figs 1 to 6D. In this embodiment, the handle 70 comprises a generally rectangular member 71 which has a first opposite pair of sides 72 which in use lie respectively on diametrically opposite sides of the tube 3. There is a second opposite pair of sides 73 which sides each include a boss or member 74 for a push fit on a respective end bar 10 of the barrel 5, there being "butterfly" recesses 75 for receiving the bar 10 of the adjacent end of the barrel 5, the bar 10 being a push fit at a waist 76 and leaving a gap at the tapering walls to provide a lost motion connection. The handle 70 is usually flat when made, the two pairs of opposite sides 72, 73 being connected by integral hinge members 77. The sides 72, 73 are thus bent out of the plane of the paper about the hinge member 77 and are offered up to the barrel, which is in the housing with the tube 3 lying over the one side 72 and under the opposite one side 72 for mounting with a push fit on the barrel, with the sides of the first pair of opposite sides being on diametrically opposed sides of the tube 3. The boss or members 74 each have a snap-fit via grooves 78, which receive ridges 79, of the end of the body 4 for enabling the handle 70 to be rotated between open and closed positions, and vice versa.

In operation, to operate the tap 1 and move it from the open position of Fig. 1C to the closed position of Fig. 1D the handle 7 is gripped manually and is lifted away from the tube 3, in the direction of the arrow 'X' in Fig. 7A, which shows schematically the operation. After the handle 7 has travelled an angular distance of 35° (180° - 145°) as shown by the arrow 'Y', the surface 13 of the segment 12 meets the bar 10 as shown schematically in Fig. 7B. Continued rotation turns the barrel 5 as the surface 13 of the segment butts against the bar 10 and the only way in which the handle 7 can continue its movement is for the barrel 5 to rotate in direction of arrow 'Z', to the closed position shown schematically in Figs. 7C and in Fig. 1D. The closed position is shown with the bar 10 extending vertically, as shown in Fig. 1D. The handle 7 cannot be moved beyond this position because the short sides of the rectangular handle 7 engage the tube 3, which acts as a stop.

It will be understood that a similar lost motion effect takes place at both ends of the barrel 5.

The reverse, or opening operation of the tap 1 takes place when the handle 7 is turned in the opposition sense, that is opposite to the arrows X, Y, Z of Figs. 7A, 7B and 7C respectively, a surface 14 of the segment 12 abutting the base 10 of the barrel 5 to effect reverse rotation after the lost motion has been taken up.

The lost motion connection 10, 12 ensures that the handle 7 effectively passes over dead centre whilst still providing an opening and closing of the tap 1.

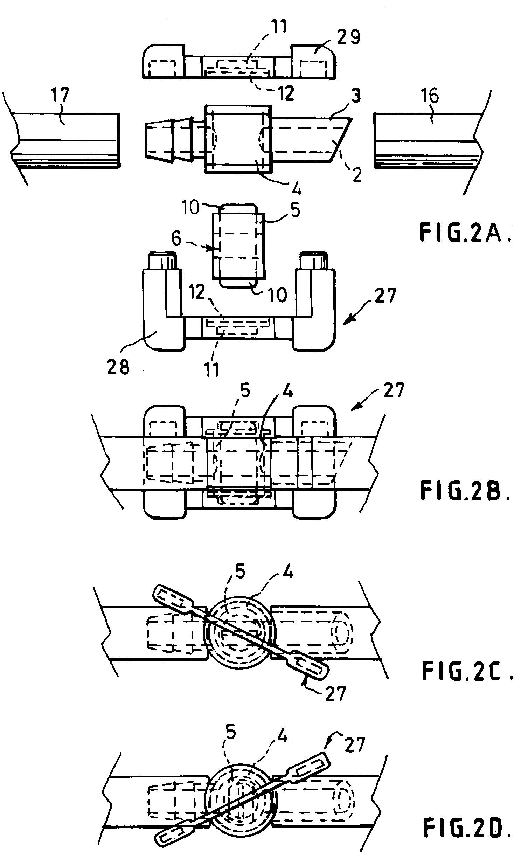

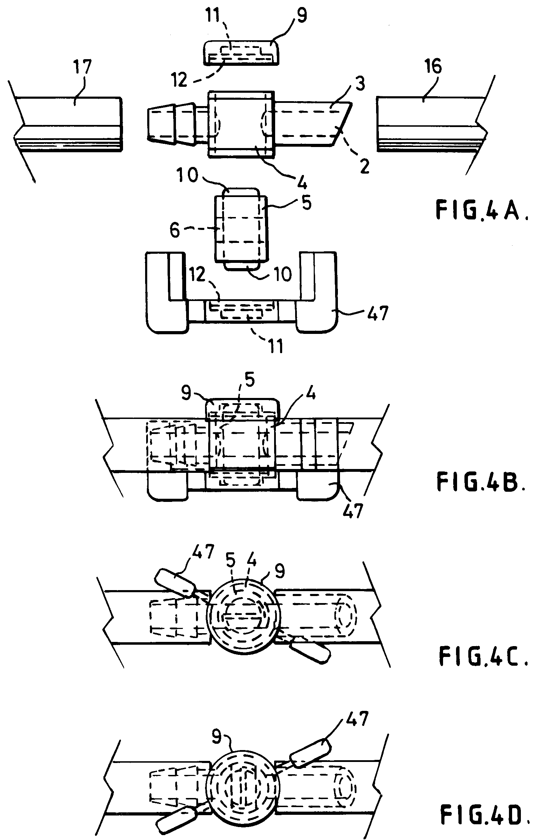

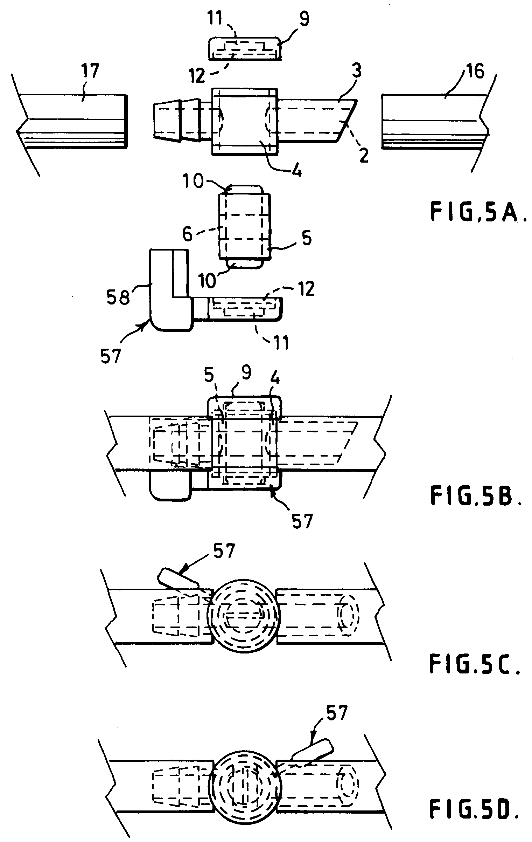

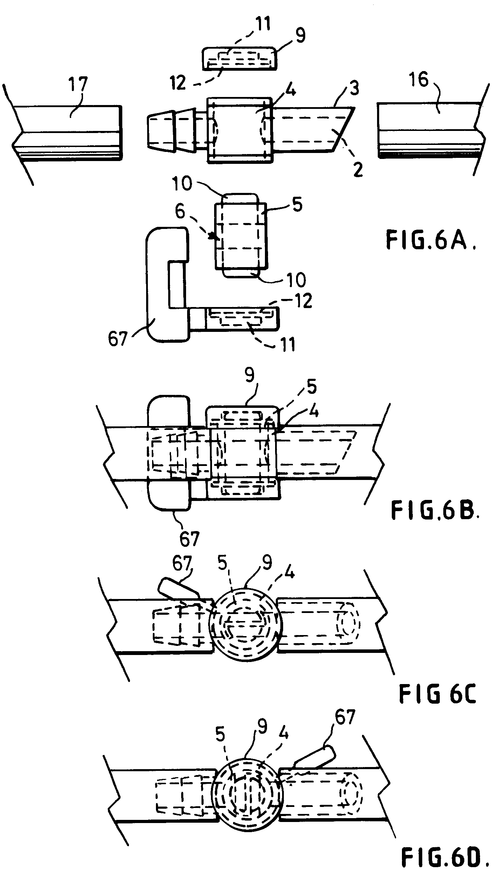

It will be understood that the construction and operation of the embodiment of taps shown in Figs. 2A to 6D are identical to that shown in Figs. 1A to 1D and 7A - 7C, the differences being in the construction of the handle 7, so the construction and operation of the further embodiments will be not repeated. Suffice it to be noted that in the embodiment of Figs. 2A to 2D, the handle 7 comprises a U-shaped handle part 27 the legs of which are connected by a push-fit connector or yoke 29, that in the embodiment 37 of Figs. 3A to 3D, the handle is a U-shaped member 38, free ends 39 of the legs being directed towards one another, there being an end cap 9 received as by a push-fit on the end of the barrel 5 opposite that on which the handle 37 is seated, that in Figs. 4A to 4D the handle 47 is again of U-shape, the free ends of the legs being flush with the outer circumference of the tube 3 and that otherwise the embodiment is like that of Figs. 3A to 3D, that the handle 57 of the embodiment of Figs. 5A to 5D has a handle which is a single L-shaped member, the free leg 58 of which terminates flush with the outer circumference of the tube 3, there again being an end cap 9, and that the embodiment of the handle 67 of Figs. 6A to 6D is of L-shape, the free end of the free leg of which projects beyond the tube 3 slightly, but in all other respects the embodiment is like that of Figs. 5A to 5D utilising again an end cap.

It will be understood that where a push-fit connection of handle or other parts is referred to, that push-fit will usually be a spigot 15 and socket 16 kind of push-fit connection.

Further, the end cap rotates with the barrel, and accordingly has a lost motion connection therewith too, of the kind shown in relation to the handles.

It will also be understood that the tube 3 may be used in a urine collection system in which for example the tube 3 is inserted in an outlet tube 16 of a leg bag for urine, and that an outlet part of the tube 3 is inserted in the inlet tube 17 of a larger urine collection bag such as one which is free-standing on a frame.

In every embodiment, a tap 1 described herein with reference to the drawings provides a simple yet effective tap which lies obliquely close to, and generally gently in contact with, the tube in both the open and the closed positions of the tap and which whilst stowed close to or in contact with the tube is nevertheless readily graspable for manual operation. Moreover, the large surface areas of the flats 13, 14 butting over a full face contact with bar 10 provide a positive driving action which is also readily engageable and disengageable without error.

It will be understood that the bar 10 may be rotated more than the 90° shown between the open and closed positions, for example 92° or 93°.

## Claims

1. A tap for controlling fluid flow along a conduit, comprising a tube, a housing in the tube, a rotatable barrel in the housing having a bore alignable with the tube longitudinal axis and being adapted in one position to close the tube against fluid flow and in a second position to open the tube to fluid flow, characterised by a handle (7) mounted on the barrel (5) and by a lost motion connection (8) between the handle and barrel whereby in both the open and closed positions the handle (7) lies closely adjacent the tube (3) and at an inclination to the longitudinal axis of the tube.

2. A tap according to Claim 1, characterised by the lost motion connection (8) comprising complementary parts of the handle (7) and barrel (5) and by an abutment (10) of one said part being adapted to be engaged by a driving member (12) of the other part.

3. A tap according to Claim 2, characterised by the complementary parts comprising a boss of the barrel carrying the abutment (10), and by a socket (11) of the handle (7) carrying the driving member (12).

4. A tap according to Claim 3 characterised by, the abutment (10) comprising a diametral bar (10) and by the driving member comprising a segment-shaped step (13) in the socket.

5. A tap according to Claim 4, characterised by the step (13) having an angle of between 90° and 180°, suitably substantially 145°.

6. A tap according to any preceding claim, characterised by the handle (7) comprising two substantially L-shaped handle parts (7a, 7b) which are mounted on the barrel (4) and which are secured together in planes laterally displaced from the axis of rotation of the barrel, suitably by complementary push-fit means (15, 16).

7. A tap according to any of claims 1 to 5, characterized by the handle (7) comprising a substantially U-shaped member (28) mounted on the barrel and by the limbs being connected by a yoke (29), suitably, by complementary push-fit means.

8. A tap according to any of Claims 1 to 6, characterised by the handle (7) comprising a substantially U-shaped member (37) mounted on the barrel suitably by the limbs thereof terminating in free ends (39) which are directed towards one another.

9. A tap according to Claim 8, characterised by the limbs (47) terminating in free ends which lie substantially in a plane at a tangent to the outer surface of the tube.

10. A tap according to any of Claims 1 to 5, characterised by the handle (7) comprising a single L-shaped member (57) mounted on the barrel by one limb.

11. A tap according to Claim 10, characterised by the other limb (58) of the L-shaped (57) member terminating at its free end in a plane lying substantially in a plane at a tangent to the outer surface of the tube.

12. A tap according to Claim 10, characterised by the L-shaped member (67) being mounted by one limb on the barrel, and by the free end of the other limb being directed towards the axis of rotation of the barrel.

13. A tap according to any preceding claim, characterised by an end cap (9) being mounted on an end of the barrel (5) opposite the handle mounting by complementary securing means, suitably push-fit means.

14. A tap according to any preceding claim, characterised by the handle (7) comprising a one piece handle with a plurality of hinge devices (77).

15. A urine drainage system, including a tap according to any preceding claim.
